# EUROPEAN PATENT SPECIFICATION

(11) **EP 0 904 070 B1**
(45) Date of publication and mention of the grant of the patent: **15.03.2000**
(21) Application number: 97927037.8
(22) Date of filing: 26.05.1997
(51) Int. Cl.: A61K 31/19, A61K 31/215

(54) **THE USE OF BUCKMINSTERFULLERENE FOR TREATMENT OF NEUROTOXIC INJURY**
VERWENDUNG VON BUCKMINSTERFULLEREN ZUR BEHANDLUNG NEUROTOXISCHER VERLETZUNGEN
UTILISATION DE BUCKMINSTERFULLERENE DANS LE TRAITEMENT DE BLESSURES NEUROTOXIQUES

(30) Priority: 03.06.1996 US 18899 P
(43) Date of publication of application: 31.03.1999
(73) Proprietor: F. HOFFMANN-LA ROCHE AG, 4070 Basel (CH)
(72) Inventor: CHOI, Dennis, Wonkyu, St. Louis, MO 63105 (US); DUGAN, Laura, Chesterfield, MO 63017 (US); LINN, Tien-Sung, Tom, Chesterfield, MO 63017 (US); LUH, Tien-Yah, Taipei, Taiwan 117 (TW)
(74) Representative: Poppe, Regina
(86) International application number: EP9702679
(87) International publication number: WO9746227

(56) References cited:
- WO-A-96/36631
- HIRSCH,A. ET AL.: "FULLERENE CHEMISTRY IN THREE DIMENSIONS:ISOLATION OF SEVEN REGIOISOMETRIC BISADDUCTS AND CHIRAL TRISADDUCTS OF C60 AND DI(ETHOXYCARBONYL)METHYLENE" ANGEWANDTE CHEMIE INT. ED. ENGL., vol. 33, no. 4, 1994, pages 437-438, XP002042666 cited in the application

## Description

The invention relates to carboxylated derivatives of the formula

C₆₀(C(COOH)₂)ₙ I

wherein C₆₀ is buckminsterfullerene and n is 1 to 4.

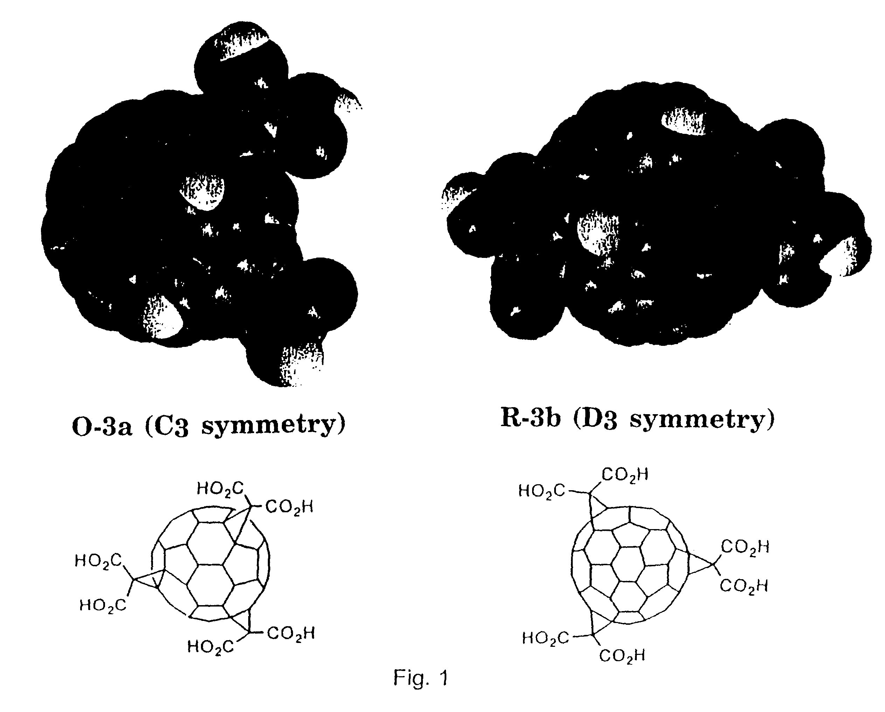

Buckminsterfullerene, C₆₀ , is a carbon sphere with alternating 5- and 6-carbon rings; the 30 carbon double bonds react easily with oxygen radicals (Science 259, 1183-1185, 1991) and so can act as a free radical scavenger. Native C₆₀, however, is soluble only in a limited number of solvents, such as toluene or benzene. The compounds useful in accordance with the present invention are water soluble carboxyfullerenes, i.e., buckminsterfullerene which has been mono- or multipli-derivativized with malonic acid, or the pharmaceutically acceptable malonic acid salts, esters and amides, where the methylene group of the malonic acid is bonded to two carbons of the fullerene sphere. The compounds useful in accordance with the present invention are thus C₆₀(C(COOH)₂)ₙ and the corresponding salts, esters and amides wherein n is an integer from 1 to 4. Examples of two isomers of C₆₀ (C(COOH)₂)ₙ where n=3 are shown in Figure 1. These compounds have been designated "O-3a" and "R-3b", and are the acids which correspond to the ethyl esters disclosed as compounds 3a and 3b in Angew. Chem. Int. Ed. Engl. 33, 437-438, 1994. The preferred compounds useful in accordance with the present invention are C₆₀(C(COOH)₂)₃ and its pharmaceutically acceptable salts, esters and amides. The most preferred compound is the acid, itself, C₆₀(C(COOH)₂)₃, especially the O-3a isomer.

It has surprisingly been found that they are biological free radical scavengers and neuroprotective agents, and thus inhibit glutamate receptor-mediated neuronal injury and serum-deprivation-induced apoptotic neuronal death.

Glutamate, the main excitatory neurotransmitter in the central nervous system, is necessary for many normal neurological functions, including learning and memory. Overactivation of glutamate receptors, however, and resulting excitotoxic neuronal injury, has been implicated in the pathogenesis of neuronal loss in the central nervous system (CNS) following several acute insults, including hypoxia/ischemia (Science 226, 850-852, 1984; Trends Pharmacol. Sci., 11, 379-387; Ann. Neurol. 19, 105-111, 1986; Science 247, 571-574, 1990); trauma (Ann. Neurol. 23, 623-626, 1988); epilepsy (Neurosci., 12, 557-567, 1984), and certain neurodegenerative disorders (Science 227, 1496-1498, 1985; Neuron 1, 623-634, 1988; Trends Pharmac. Sci. 11, 379-387, 1990; Neurol. 40, 32-37, 1990; Ann. Neurol. 31, 119-130, 1992).

Oxidative stress, caused by reactive oxygen species, represents another injury mechanism implicated in many of the same acute and chronic diseases (Stroke 9, 445-447, 1978; Proq. Brain Res. 63, 227-235, 1985; J. Neurosurq. 64, 803-807, 1986; Cerebrovas. Brain Mezab. Rev. 1, 165-211, 1989; Free. Radic. Biol. Med. 6, 289-301, 1989; J. Neurochem. 59, 1609-1623, 1992). Reactive oxygen species (e.g., superoxide radical) would cause oxidative damage to cellular components, such as peroxidation of cell membrane lipids, inactivation of transport proteins, and inhibition of energy production by mitochondria.

These two events, glutamate excitotoxicity and oxidative stress, may be interlinked; reactive oxygen species formation may occur as a direct consequence of glutamate receptor overstimulation (Soc. Neurosci. Abs. 18, 756, 1992; Nature 364, 535-537, 1993) and thus mediate a component of glutamate neurotoxicity (Neuron. 1, 623-634, 1988; Science 262, 689-694, 1993). Excitotoxicity, in turn, can be reduced by free radical scavengers, including Cu, Zn-superoxide dismutase and catalase (J. Neurochem. 49, 1222-1228, 1987; Acta Neurochirurgica 51, 245-247, 1990), the 21-aminosteroid "lazaroids" (Neuron 5, 121-126, 1990), the vitamin (J. Neurochem. 49, 1222-1228, 1987; the 21-aminosteroid "lazaroids", the vitamin E analog, trolox (Brain Res. 639, 102-108, 1994), spin-trapping agents such as phenybutyl-N-nitrone (Brain Res. 574, 193-197, 1992), and the ubiquinone analog, idebenone (Neurosci. Lett. 178, 193-196, 1994) which reduce the amount of reactive oxygen species.

Free radical scavengers are neuroprotective in *in vitro* as well as *in vivo* models of traumatic or hypoxic/ischemic CNS injury. N-methyl-D-aspartate and AMPA/kainate receptor antagonists are neuroprotective in oxygen-glucose deprivation injury *in vitro* (Neuron 1, 623-634, 1988; J. Neurosci. 13, 3510-3524, 1993), and reduce loss of brain tissue in animal models of ischemia (Science 226, 850-852, 1984; Science 247, 571-574, 1990). Free radical scavengers also protect against excitotoxic neuronal death *in vitro (J.* Neurochem. 49, 1222-1228, 187; Neuron 5, 121-126, 1990), and reduce ischemic injury *in vivo* (Amer. J. Physiol. 256, H589-593; Stroke 21, 1312-1317, 1990; Stroke 22, 896-901, 1991; Free Rad. Biol. Med. 12, 29-33, 1992). Transgenic animals which overexpress the free radical scavenger enzyme, CuZn superoxide dismutase (SOD), are resistant to glutamate toxicity (Acta Neurochirurgia 51, 245-247, 1990), and ischemic brain injury (Ann. Neurol. 29, 482-486; Proc. Natl. Acad. Sci., USA 88, 11158-62, 1991).

Programmed cell death, or apoptosis, also contributes to cell death in certain neurologic disease states. For example, apoptosis would mediate delayed neuronal degeneration days after ischemia-reperfusion (J. Neurochem. 61, 1973-1976, 1994; Neuroreport 5, 493-496, 1994), and would be a factor in neuronal cell death in certain neurodegenerative diseases (Neurosci. Lett. 170, 191-194, 1994). Oxidative stress due to free radical oxygen species would be one of the insults that can trigger apoptosis (Nature 356, 397-400, 1992; Neurotoxicol. 15, 81-91, 1994; J. Natl. Cancer Inst. 86, 1286-1295, 1994), so that free radical scavengers would also be able to limit programmed cell death (J. Neurochem. 62, 376-379, 1994; Neurosci. Abs. 20, 432, 1994). Bcl-2 appears to act on a free radical scavenging pathway to mediate its cytoprotective effects against apoptosis (Cell. 75, 241-251, 1993).

The main object of the present invention is the use of carboxylated derivatives of C₆₀(C(COOH)₂)ₙ, wherein C₆₀ is buckminsterfullerene and n is an integer from 1 to 4, for the manufacture of a medicament for the treatment or prophylaxis of diseases caused by free radicals, especially when the free radicals are released as a result of glutamate neurotoxicity. Treating neurotoxic injury within the meaning of the present invention means reducing the extent of damage to central neurons surrounding a central neuron which has released glutamate due to its having been damaged by a neurotoxic event. Neurotoxic events include acute neurological insults such as hypoxia/ischemia, such as occurs during stroke, hypoglycemia, epilepsy or trauma. Neurotoxic events may also be chronic neuronal damage caused by neurodegenerative disorders such as Huntington's disease, Alzheimer's disease, amyotropic lateral sclerosis ("ALS"), and the neurodegenerative effects of AIDS.

A further object of the present invention is the use of buckminsterfullerene for the manufacture of a medicament for inhibiting neurotoxic injury in patients where said injury is caused by the metabolism of arachidonic acid released by neurons due to stimulation by glutamate of NMDA receptors of said neurons.

Arachidonic acid ("AA") is released in neurons due to an influx of excessive Ca²⁺ into the neuronal cells which is caused by NMDA receptor stimulation by glutamate (the glutamate having been released by neurons which were damaged by the neurotoxic event, itself). The excessive Ca²⁺ influx activates phospholipase A₂, a calcium-dependent enzyme which breaks down cell membranes liberating the AA. The metabolism of AA by endogenous lipoxygenases and cyclooxygenases leads to the production of the oxygen free radicals that trigger peroxidative degradation of neuronal lipid membranes (Acta Physiol. Scand. 492, 121-128, 1980; Proq. Brain Res. 63, 227-232, 1985) which results in the neuronal damage or death. Therefore, in accordance with the present invention, reducing oxygen-derived free radicals by administering a composition comprising a free radical scavenging carboxyfullerene described herein provides an alternative mechanism by which glutamate-induced neurotoxicity is inhibited.

The preferred object of the invention comprises the use of the above mentioned compounds for the manufacture of a medicament for treating stroke. In accordance with the present invention, stroke is defined as an acute neurotoxic event in the brain of a patient wherein the neurotoxic event occurs due to a loss of blood flow to neurons of the brain.

The carboxyfullerene compounds described herein are administered systematically as a medicament containing the active compound and a pharmaceutically acceptable carrier compatible with said compound. In preparing such a medicament, any conventional pharmaceutically acceptable carrier may be utilized. When the drug is administered orally, it is generally administered at regular intervals.

In therapeutic use, the compounds useful in accordance with the invention may be administered by any route whereby drugs are conventionally administered. Such routes include intravenously, intramuscularly, subcutaneously, intrathecally, intraperitoneally, topically, as well as orally. Preferably, the method of the invention is carried out via oral or intravenous routes of administration.

The medicament can be made up in any conventional form, including a solid form for oral administration such as tablets, capsules, pills, powders, granules, and the like. The medicament may be sterilized and/or may contain adjuvants such as preservatives, stabilizers, wetting agents, emulsifiers, salts for varying the osmotic pressure, and/or buffers.

Typical preparations for intravenous administration would be sterile aqueous solutions including water/buffered solutions. Intravenous vehicles include fluid, nutrient and electrolyte replenishers. Preservatives and other additives may also be present such as antibiotics and antioxidants. Medicaments for bolus i.v. administration may contain up to 10 mg/ml (10,000 mg/liter) of a carboxyfullerene described herein. Medicaments for drip i.v. administration preferably contain from about 50 mg/liter to about 500 mg/liter of a carboxyfullerene described herein.

In accordance with this invention, the carboxyfullerenes described herein are useful in pharmaceutically acceptable oral modes. These medicaments contain said compound in association with a compatible pharmaceutically acceptable carrier material. Any conventional carrier material can be utilized. Any conventional oral dosage form such as tablets, capsules, pills, powders, granules, and the like may be used. The carrier material can be an organic or inorganic inert carrier material suitable for oral administration. Suitable carriers include water, gelatin, gum arabic, lactose, starch, magnesium stearate, talc, vegetable oils, polyalkylene-glycols, petroleum jelly and the like. Furthermore, the medicament may contain other pharmaceutically active agents. Additional additives such as flavoring agents, preservatives, stabilizers, emulsifying agents, buffers and the like may be added in accordance with accepted practices of pharmaceutical compounding.

A preferred oral dosage form comprises tablets, capsules of hard or soft gelatin, methylcellulose or of another suitable material easily dissolved in the digestive tract. The oral dosages contemplated in accordance with the present invention will vary in accordance with the needs of the individual patient as determined by the prescribing physician. The preferred oral dosage form is capsules or tablets containing from 50 to 500 mg of a carboxyfullerene useful in accordance with the present invention.

In carrying out the invention, a compound useful in accordance with the invention is generally given to adults daily, preferably orally or intravenously, in an amount of from about 1.5 mg/kg to about 1500 mg/kg daily, in single or divided doses, preferably from about 10 mg/kg to about 60 mg/kg daily, with the precise dosage being varied depending upon the needs of the patient. In general, this therapy is carried out for a period of about, three months. Alternatively, the method of the invention may be carried out prophylactically for an indefinite time in those patients who are have a high risk of suffering an acute neurotoxic event, such as a stroke. For the treatment of an acute neurotoxic event, the patient should be treated in accordance with the method of the invention as soon as possible after the diagnosis of the acute neurotoxic event, preferably within twelve hours, and most preferably within six hours, of the onset of the neurotoxic event.

The Figures 1-11 demonstrate the invention in more detail.

**Fig. 1** Structures of hexacarboxyfullerene, C₆₀(C(COOH)₂)₃, isomers O-3a and R-3b.

**Fig. 2** Spectrum of purified C₆₀(C(COOH)₂)₃ malonic ester (O-3a enantiomer) by proton NMR spectroscopy.

**Fig. 3** Spectrum of purified C₆₀(C(COOH)₂)₃ malonic ester (O-3a enantiomer) by fast-atom bombardment mass spectrometry.

**Fig. 4** Electron paramagnetic resonance spectra demonstrating the free radical scavenging activity of C₆₀(C(COOH)₂)₃ against H₂O₂ (A: untreated, B: treated with the O-3a enantiomer) and superoxide radical O₂^{•-} (C: untreated, D: treated with the O-3a enantiomer, E: treated with the R-3b enantiomer). The arrows point to an artifactual signal generated by a contaminant in the EPR cavity.

**Fig. 5** Neurotoxicity produced by exposure of cultured neurons to NMDA without treatment and with three concentrations of C₆₀(C(COOH)₂)₃.

**Fig. 6** Neurotoxicity produced by exposure of cultured neurons to AMPA without treatment and with four concentrations of C₆₀(C(COOH)₂)₃.

**Fig. 7** NMDA-stimulated accumulation of tracer ⁴⁵Ca²⁺ in cultured neurons with and without the co-application of C₆₀(C(COOH)₂)₃.

**Fig. 8** Apoptotic neuronal cell death produced in glial-deficient cultures by serum deprivation without treatment and with two concentrations of C₆₀(C(COOH)₂)₃.

**Fig. 9** EPR spectra comparing the free radical scavenging activity of two C₆₀(C(COOH)₂)₃ isomers.

**Fig. 10** Comparison of two C₆₀(C(COOH)₂)₃ isomers in protecting neurons from injury produced by application of NMDA (A) and AMPA-induced neuronal death (B). EPR spectra of spin-labeled lipids (5- or 16-doxyl ketostearic acid) (C incoporated into lipids from mouse brain, plus either of the two C₆₀(C(COOH)₂)₃ isomers.

**Fig. 11** Survival curves for FALS mice treated with intraperitoneal mini-osmotic pumps containg saline or 15 mg/ kg/ day carboxyfullerene.

The data herein demonstrates that the disclosed carboxyfullerenes are a novel class of antioxidants with the ability to scavenge multiple oxygen-derived free radicals, and that these compounds have unusual broad and powerful neuroprotective capabilities, attenuating neuronal death due to glutamate excitotoxicity, and apoptosis.

### Examples

### Solutions

Media stock (MS) consists of Eagle's Minimal Essential Media with 25 mM glucose, minus L-glutamine.

Plating medium consists of MS supplemented with L-glutamine (2 mM), 5% fetal calf serum, and 5% horse serum.

Growth medium contains MS, 10% horse serum, and 2 mM L-glutamine.

Brief exposure to NMDA is carried out in HEPES-buffered balanced salt solution, pH 7.40 (HBBSS), containing, in mM, 116 NaCl, 5.4 KCl, 0.8 MgSO₄, 1.8 NaPO₄, 12 HEPES, 25 NaHCO₃, 5.5 D-glucose, with 10 (M L-glycine.

Balanced salt solution (BSS) was, in mM, 116 NaCl, 5.4 KCl, 0.8 MgSO₄, 1.8 NaPO₄, 26.2 NaHCO₃, and 5.5 D-glucose.

The original isomer of C₆₀(C(COOH)₂)₃ was the O-3a isomer, which represents the major product of the synthesis. Stock of this compound was prepared as a 25 mM solution in water. Stocks were used within 72 hours of preparation, and were stored at -20°C in the dark.

Unmodified C₆₀ was dissolved in toluene to make a 50 mM stock.

### Cortical Cell Cultures

Mouse neocortical cultures were prepared as neuron-astrocyte co-cultures (approximately 50% astrocytes) (Rose et al., 1992) or as neuronal cultures (< 2% astrocytes). Mouse embryos (gestational day 15) are removed from pregnant anesthetized Swiss-Webster mice, and the neocortex is dissected away from other brain structures. After brief incubation in trypsin, the cells are dissociated by trituration, and the cell suspension is then diluted into plating medium and plated onto previously-prepared 24-well Plastek culture plates coated with poly-D-lysine/laminin (for neuronal cultures) or onto an existing bed of astrocytes (for co-cultures). After 1-2 days *in vitro,* a partial exchange with glial conditioned medium is made for "pure" neuronal cultures, and cytosine arabinoside (3µM) is added immediately after feeding to inhibit glial proliferation. Mixed cultures are fed biweekly with growth medium until 11-12 days in vitro, when they are fed with serum-free MS containing 2 mM L-glutamine. Unless otherwise stated, cells were used at 14-16 days *in vitro.*

### Example 1

### Synthesis and properties of carboxyfullerenes (2,2-fulleromalonic acids)

Diethyl 2,2-fulleromalonic ester C₆₀ (1 g, 1.39 mmol) was added to freshly distilled toluene (1000 ml) and stirred for a few minutes to obtain a clear, violet solution. Diethyl bromomalonate (0.474 ml, 2.78 mmol) was added dropwise to the stirred solution. Addition of 0.526 ml (3.475 mmol) DBU (1,8-diazobicyclo[5,4,0]-undec-7-ene) to the fullerene solution caused the color to change from violet to dark red. After stirring overnight in air at room temperature, the solution was filtered to remove the ammonium salts, and the solvent was evaporated in varus. The residue was dissolved in a small volume of chloroform, and added to the top of a silica gel column (packing material was 70-230 mesh or 230-400 mesh flash chromatographic gel from Merck). The fullerene products were eluted using a gradient from toluene/hexane (1:1) to toluene (100%). The initial elution gave 5 fractions, unreacted fullerene, monoadducts, bisadducts, trisadducts, multiadducts. Solvent of each fraction was removed *in vacuo* and the solid residue was chromatographed repeatedly until pure. The fractions containing the bisadducts and the trisadducts each gave two main products on reseparation.

2,2-fulleromalonic acid(s) Samples containing single isomers of the ester adducts (100 mg) were dissolved in toluene (50 ml) under nitrogen. A 20:1 molar excess of NaH was then added. After stirring for 2-3 h at 100 °C, 1 ml of methanol was added dropwise to the hot solution, producing a vigorous evolution of gas. The quantitative precipitation of sodium salt occurred concurrently. The salt was collected by centrifugation, and dried under vacuum. The dried compound was washed with 2 M H₂SO₄, then water. The product, representing the malonic acid derivative of the corresponding ester, was dried under vacuum overnight to give a fine brown powder.

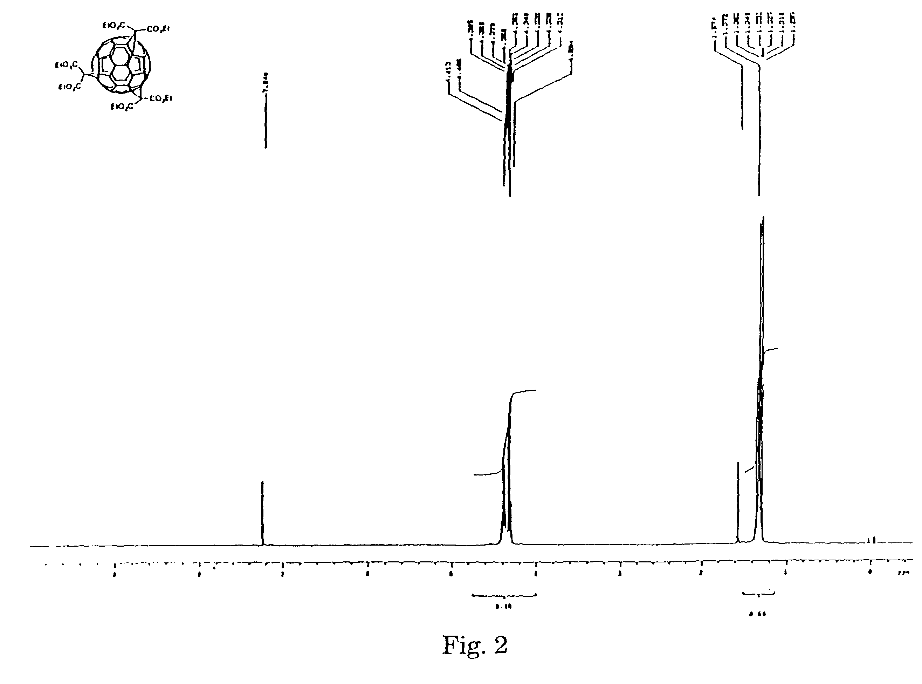

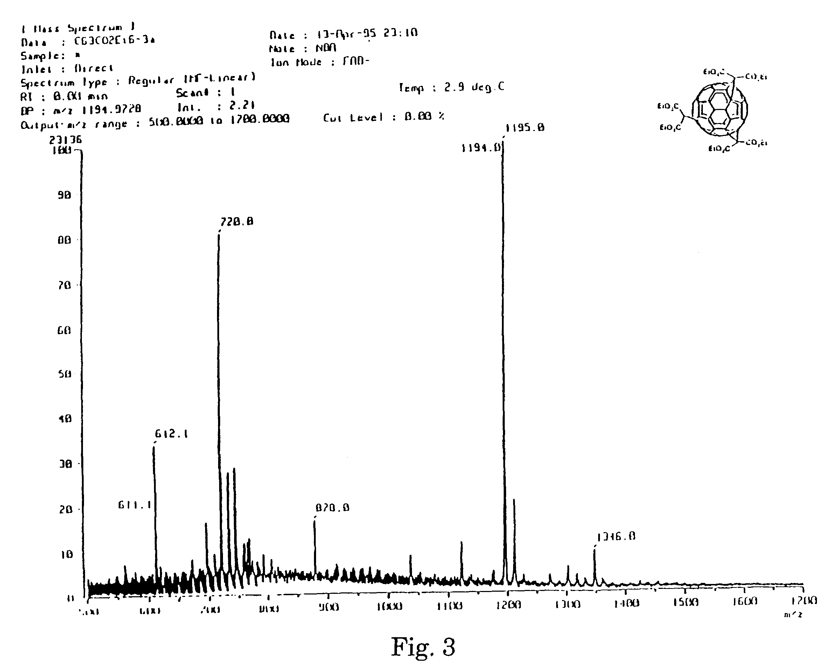

Mass spectroscopy was performed on a sample containing the O-3a enantiomer of trisadducted fullerene ester; this ester was then hydrolyzed and acidified to form the final carboxyfullerene compound, C₆₀(C(COOH)₂)₃. Proton NMR and mass spectra (Figs. 2 and 3, respectively) of the purified C₆₀ ester indicate that the sample contains a single isomer of 2,2-fulleromalonic ester (O-enantiomer with mass = 1194/1195) with a small amount of C₆₀ (mass = 720) which may have arisen from fragmentaion of the adduct. Results from the original compound, the O-3a enantiomer, are described herein. More recent studies with the other enantiomer, R-3b, indicate that it is also an effective neuroprotectant.

Solutions of carboxyfullerene were translucent brown. However, a concentrated (50 mM) solution filtered through 0.45 (m nylon filters did not leave any residue on the filters, indicating a true solution. Control experiments verified that the compounds did not interfere with the colorimetric LDH assay. Solutions of up to 25 mM of the carboxyfullerenes failed to alter the pH of the experimental solutions.

### Example 2

### Excitatory amino acid toxicity and application of fullerenols

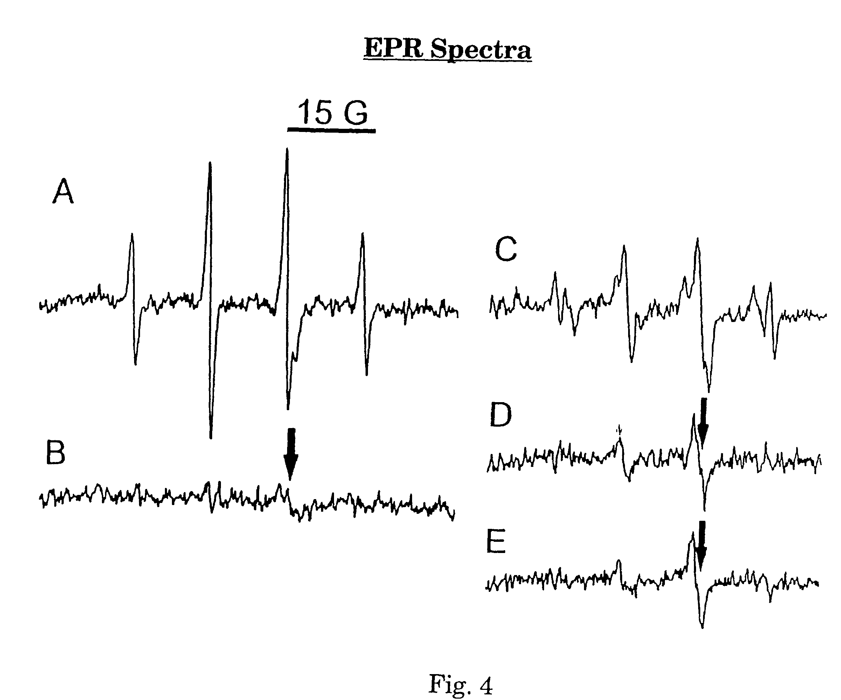

By electron paramagnetic resonance spectroscopy, C₆₀(C(COOH)₂)ₙ compounds proved to be a potent free radical scavengers, capable of eliminating both hydroxyl and superoxide radials. Fig. 4 shows EPR spectra A - E demonstrating the free radical scavenging activity of C₆₀(C(COOH)₂)₃. Spectra A and B, respectively, show the hydroxyl radical (spin adduct: DMPO-OH⁽) generated by H₂O₂ over a 15 minute period, and the elimination of this OH signal when 150 (M O-3a isomer was included with the H₂O₂. Superoxide radical (O₂⁽⁻) was also effectively scavenged by C₆₀(C(COOH)₂)ₙ compounds. O₂⁽⁻, which forms the spin adduct DMPO-OOH, was generated by incubating xanthine oxidase with xanthine (Spectrum C). Co-incubation with either 40 (M O-3a isomer or 40 (M R-3b eliminated the superoxide radical signal (Spectra D and E, respectively). EPR analysis of other water-soluble C₆₀(C(COOH)₂)ₙ compounds with n=1 or 2 confirmed that they were also effective free radical scavengers (data not shown).

The hexacarboxylated compound, C₆₀(C(COOH)₂)₃, which was soluble in water to at least 50 mM, protected cortical neurons against excitotoxic injury produced by exposure to N-methyl-D-aspartate (NMDA) and (-amino-3-hydroxy-5-methyl-4-isoxazole propionic acid (AMPA). The dicarboxy and tetracarboxy fullerene derivatives were less soluble in aqueous solution and, while possessing neuroprotective activity, were less effective neuroprotectants than the hexacarboxy derivative.

Carboxyfullerene (3-300 (M) was co-applied to neuron-astrocyte co-cultures described above with NMDA for assessment of neuroprotection. Brief exposure to NMDA in the presence of the carboxyfullerene was carried out by triple exchange of the medium with HBBSS, followed by addition of 50µM- 500µM NMDA plus carboxyfullerene for 10 minutes. The NMDA and carboxyfullerene were removed by quadruple exchange of the medium with MS, and the cultures were placed in the humidified, CO₂ (5%), 37°C incubator for 24 hours, when the extent of injury was assessed.

Prolonged (24 hours) exposure to 5-100 (M AMPA is routinely used to produce cortical neuronal injury. In another series of experiments, carboxyfullerene (3-300 (M) was co-applied to neuron-astrocyte co-cultures described above, with AMPA, for assessment of neuroprotection. After the medium was exchanged twice with MS, AMPA and carboxyfullerene were added and cultures were returned to the 37°C incubator. The NMDA receptor antagonist, MK-801 (10 (M) was included with the AMPA and carboxyfullerene to eliminate secondary activation of NMDA receptors by endogenous glutamate release.

Neuronal death was assessed at 24 hours after NMDA or AMPA administration by phase contrast microscopy at 200-400x, and by measurement of lactate dehydrogenase (LDH) efflux from dying cells into the bathing medium (Koh and Choi, 1987). Quantitation of cell death was confirmed in some experiments by trypan blue or propidium iodide staining and cell counting.

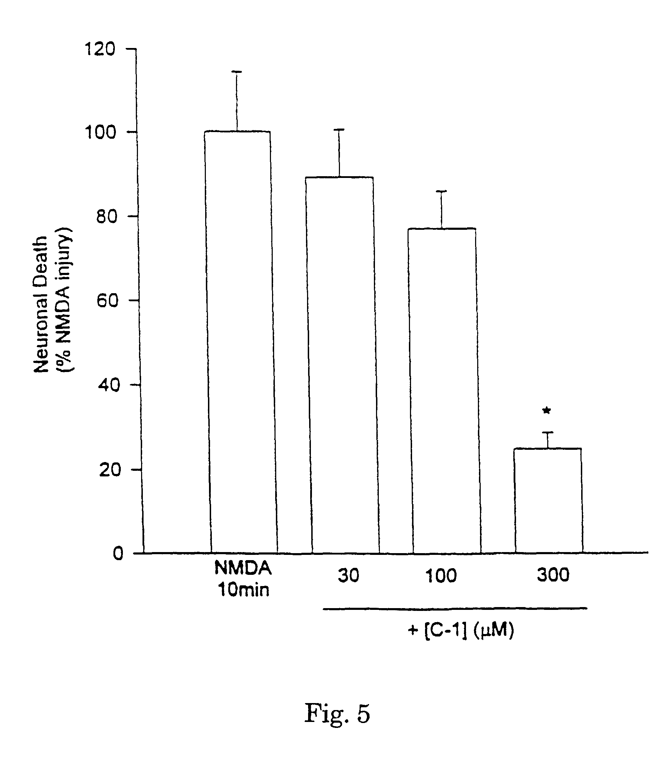

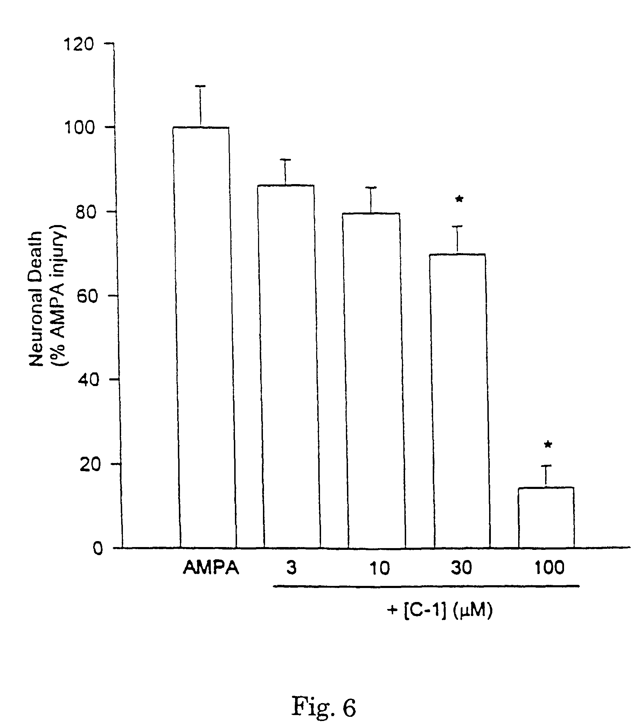

Results for C₆₀(C(COOH)₂)₃ are shown in Figures 5 and 6. C₆₀(C(COOH)₂)₃ showed robust neuroprotection against NMDA (Fig. 5) and AMPA-induced (Fig. 6) excitotoxic neuronal injury. C₆₀(C(COOH)₂)₃ (300(M) reduced neuronal death from NMDA by at least 60% in all experiments performed to date, and afforded nearly complete protection in some experiments. The amount of LDH present in washed controls was subtracted to give the signal specific to NMDA toxicity. Data are graphed as the percentage of cell death produced by NMDA alone: this exposure killed 51 ( 9, 44 ( 6, and 88 ( 14 % of the total neurons per culture, mean ( S.D. * = p<0.05 vs. NMDA, by ANOVA followed by Student-Newman-Keuls test for multiple comparisons. Pooled data from three experiments (Fig. 5) demonstrate a 75% reduction in neuronal cell death.

C₆₀(C(COOH)₂)₃ reduced neuronal cell death from AMPA by >80% at 100 (M (Fig. 6). MK-801 (10 (M) was included with AMPA to eliminate secondary activation of NMDA receptors by released endogenous glutamate. Values are mean ( SEM, n=3-4/experiment, data from 4 experiments are pooled. Data are graphed as the percentage of cell death produced by AMPA/MK-801 alone, representing 40-70% of the total neurons/culture. * = p<0.05 vs. AMPA, by ANOVA followed by Student-Newman-Keuls test for multiple comparisons.

The experiments described here are, to our knowledge, the first use of these compounds as cytoprotective agents or as antioxidants.

### Example 3

### ⁴⁵Ca²⁺ tracer experiments

To verify that protection against NMDA-induced neuronal cell injury was not due to a reduction in NMDA-induced calcium influx, ⁴⁵Ca²⁺ tracer studies were performed. Cultures were washed twice with HBBSS and then exposed to NMDA (300 (M), either alone or with C₆₀(C(COOH)₂)₃, in HBBSS containing tracer ⁴⁵Ca²⁺ (0.5 (Ci/culture well; NEN, Boston, MA). The exposure was terminated after 10 minutes by exchanging the medium four times with unlabelled HBBSS, followed by lysis of the cells by addition of 0.2% sodium dodecyl sulfate (SDS). The cells were left in SDS for 2 hours, and the lysate was then transferred to scintillation vials. An additional wash of each culture well with SDS was added to the vial, which then underwent (-counting.

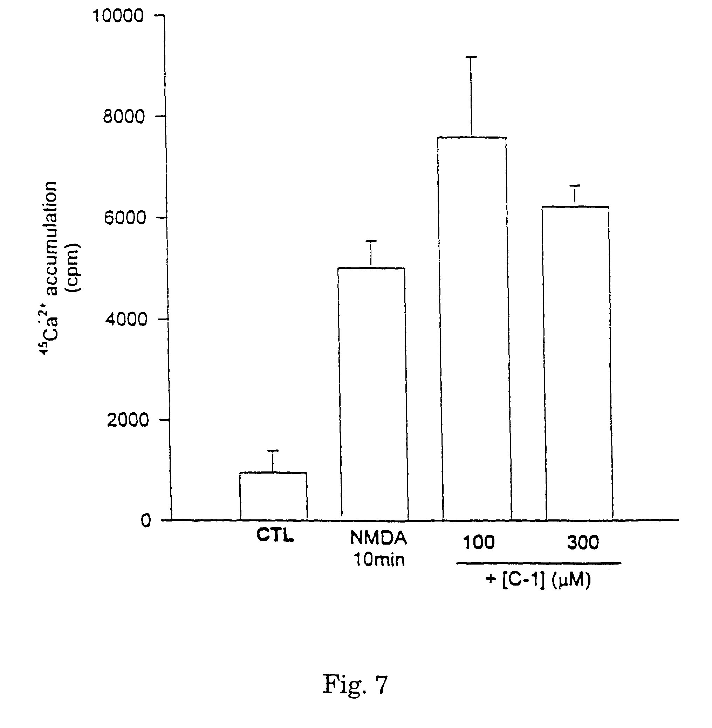

The results in Figure 7 demonstrate that the carboxyfullerenes are not NMDA receptor antagonists. ⁴⁵Ca²⁺ accumulation was not affected by co-application of up to 300 (M of C₆₀(C(COOH)₂)₃ with NMDA. Basal ⁴⁵Ca²⁺ was subtracted from all conditions to give the ⁴⁵Ca²⁺ increase specific to NMDA receptor activation. Mean ( SEM, n=4.

### Example 4

### Serum deprivation apoptotic neuronal death

Cortical neurons in glia-deficient culture undergo apoptotic cell death 24-48 hours after removal of serum, as characterized by morphologic changes such as cell body shrinkage, fragmentation of neuronal processes, and chromatin condensation by Hoechst 33258 staining (Dugan et al, 1995). Apoptotic neuronal death due to serum deprivation was also attenuated by co-application of hexacarboxyfullerene. This injury has been shown to have features typical of apoptosis, including DNA laddering, chromatin clumping, cell shrinkage, formation of apoptotic bodies, and protection by macromolecular synthesis inhibitors. Cortical neurons in cultures containing less than 1 % astrocytes were deprived of serum by exchanging the serum-containing growth medium with a balanced salt solution supplemented with amino acids (excluding glutamine). Washed controls were returned to medium containing 2% fetal calf serum, 2% horse serum in BSS. After 24 and 48 hours, the cells were photographed using phase contrast optics at 100-400X, using a Nikon inverted microscope.

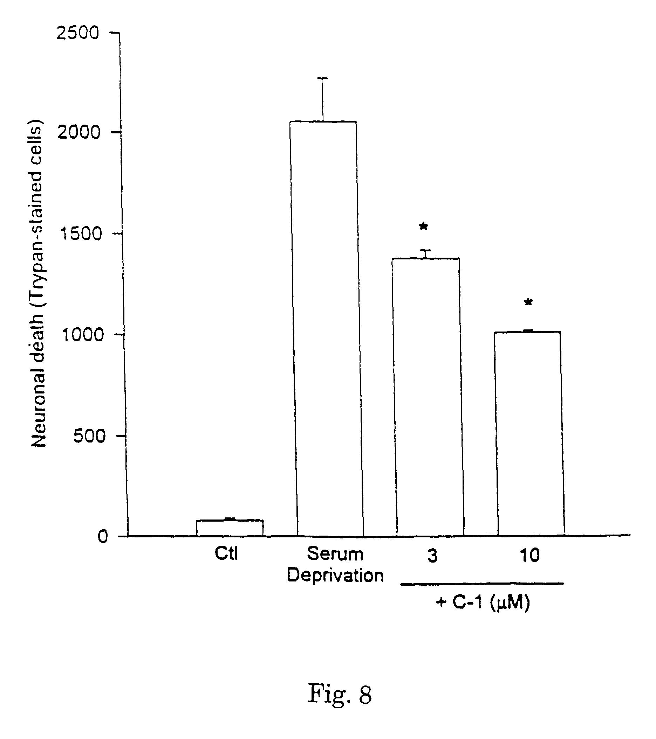

Additionally, neuronal cell death was determined at 48 hours removal of serum by counting cells that are no longer able to exclude trypan blue. As shown in Figure 8, neuronal cell death, determined at 48 hours after the onset of serum-deprivation by counting cells that were no longer able to exclude trypan blue, demonstrated a significant reduction in neuronal death. Cells treated with 10 (M C₆₀(C(COOH)₂)₃ demonstrated a 50% reduction in neuronal death. Washed control cultures maintained in serum-containing medium had very little death. Values are mean ( SEM, n=3-4/experiment. This experiment is representative of 3 experiments. * = p<0.05 vs. serum-deprivation, by ANOVA followed by Student-Newman-Keuls test for multiple comparisons.

### Example 5

### Polar location of carboxyl groups on C₆₀ improves neuroprotective efficacy

Two regioisomers of C₆₀C(COOH)₂)₃, O-3a and R-3b, were synthesized and purified following the method of Lamparth and Hirsch (Lamparth and Hirsch (1990)). The purity of these compounds was verified by NMR and UV/Visible spectral analyses. In the O-3a compound, all methano bridges are in e(quatorial) positions relative to each other (e,e,e), and the molecule has C₃ symmetry as demonstrated ¹H and ¹³C NMR spectroscopy. Compound R-3b has methano bridges which lie on a belt along the equator about the threefold axis of the C₆₀ framework in trans-3 positions (trans-3, trans-3, trans-3), and has D₃ symmetry. The 3-D structures shown in Figure 1 illustrate the polar distribution of the carboxyl groups on O-3a and the equatorial distribution of the carboxyl groups on R-3b.

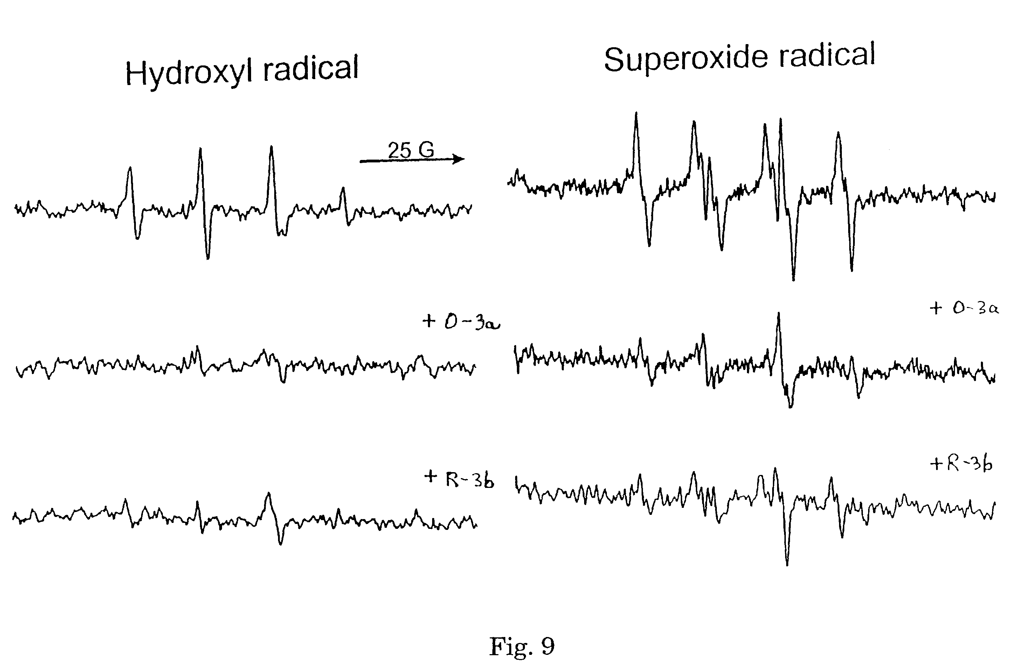

Figure 9 shows that, chemically, these two isomers showed similar abilities to scavenge ^{•}OH and O₂^{•-} free radicals, as judged from spin-trap/EPR spectra. Figure 9 shows the EPR spectra of ^{•}OH (from 100 µM H₂O₂ in Fe²⁺ by the Fenton reaction) and O₂^{•-} (from xanthine + xanthine oxidase) radicals with 100 mM 5,5-dimethyl-1-pyrolline-N-oxide (DMPO) as the spin-trapping agent. Hydroxyl radical (spectra on left): ^{•}OH in the presence of DMPO alone (top), in the presence of 4 µM O-3a (middle), or in the presence of 4 µM R-3b (bottom). Superoxide radical (right): O₂^{•-} in DMPO alone (top), in the presence of 400 µM O-3a (middle), in the presence of 400 µM R-3b (bottom). The arrow designates a spurious signal due to an unknown radical in the cavity. The sample was analyzed in a quartz flat cell (60 x 10 x 0.25 mm) in a Bruker 200, X-band EPR spectrometer. Settings were power = 1.6 mW, modulation = 1 G, field modulation = 100 Hz, R.G. = 3.2x10⁵.

The EPR results demonstrat that both O-3a and R-3b isomers are extremely potent scavengers of ^{•}OH at concentrations 100 to 1000-fold less than reported for most other free radical scavengers. The scavenging ability of carboxyfullerene towards ^{•}OH is 10-fold greater than than its potency for O₂^{•-} radicals.

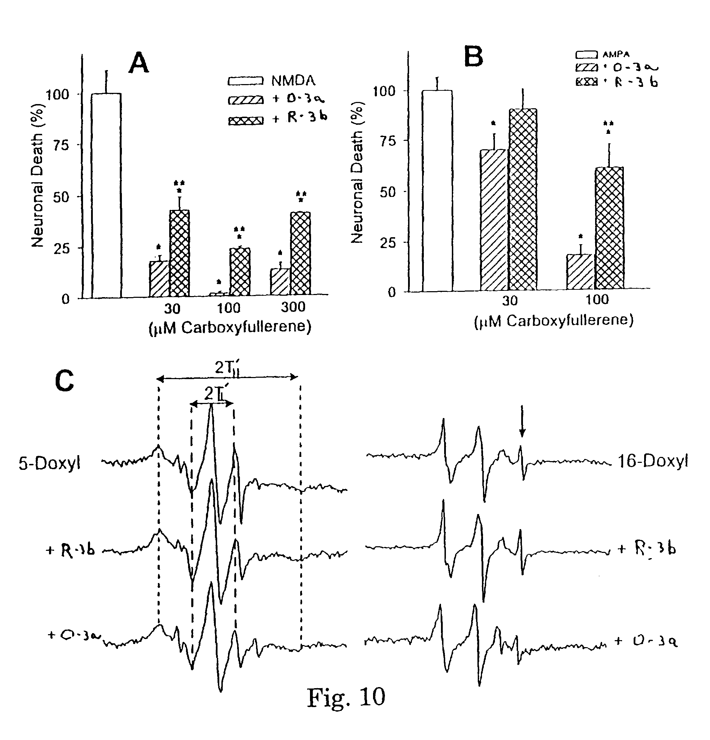

Although the O-3a and R-3b isomers are equipotent antioxidants by EPR analysis, the O-3a compound was biologically more effective than R-3b against both NMDA and AMPA receptor-mediated injuries. These results are shown in Fig. 10. Figure 10 demonstrates that O-3a provided slightly better protection from injury produced by application of NMDA (A), but substantially better protection against AMPA-induced neuronal death (B). Values are mean ± SEM, n=8-12 per conditions. * = p<0.05 vs. untreated injury condition, using ANOVA followed by Student-Newman-Keuls test for multiple comparisons. ** = p<0.05 R-3b different than O-3a. Fig. 10 also shows the EPR spectra of spin-labeled lipids (C) (5- or 16-doxyl ketostearic acid) incoporated into lipids from mouse brain, plus either O-3a or R-3b. The spin labels were added to lipids extracted from adult mouse brain at a ratio of 1:100. EPR settings were the same as those listed in the legend for Figure 9. Both isomers produce a shift in the order parameter (S) of the 5-doxyl group, but O-3a produced a greater change in S (Table 1). O-3a also altered the liquid state signal from 16-doxyl ketostearic acid to a greater extent than R-3b, indicating that O-3a increased the disorder, or fluidity, of the lipids (Table 1). Both results suggest that O-3a enters the lipid bilayer to a greater extent than R-3b.

In addition to its greater activity in protecting neurons from NMDA and AMPA receptor-mediated injuries, O-3a provided enhanced protection over R-3b from such injury in endothelial cell cultures and in hepatocytes. This difference was apparently due to the ability of the O-3a isomer to enter membranes to a greater extent than R-3b. The polarity of O-3a enables it to enter the cell membrane more easily. This ability to intercalate into membranes suggests that O-3a would provide better protection of cell membranes from lipid peroxidation than R-3b. Thus, the location of functional groups (polar vs. circumferential) is important for the protective efficacy of C₆₀ derivatives.

**Table 1.**

| Membrane parameters from spin-label/EPR experiments | | | |
|---|---|---|---|
| Compound | Order parameter^{a} | Correlation time^{b} | Lipid/aqueous partition factor^{c} |
| O-3a | 0.84 ± 0.01 | 5.7 ± 0.5 ns | 1.0 ± 0.2 |
| R-3b | 0.86 | 4.5 | 0.6 |
| Control | 0.88 | 5.1 | 0.8 |

| | | | |
|---|---|---|---|
| ^{a} Order parameter, S = a(T_{/}'-T_{⊥}')/(T_{/} -T_{⊥}), where T_{/}' is the measured hyperfine splitting for the parallel orientation in 5-doxylstearic acid spin label/phospholipids, and T_{⊥}' the perpendicular orientation. We assume a = 1, and T_{/} - T_{⊥} = 25 G, | | | |
| ^{b} Correlation time, τ_{c} = 6.5 x 10-10 wₒ[(hₒ/h₋₁)-1], where wₒ is the width of the mid-field line in gauss, hₒ and h₋₁ are peak height of mid- and high-field lines on the first derivative absorption measured for 5-doxylstearic acid. | | | |
| ^{c} Lipid/aqueous partition factor, *f* = h_{L}/h_{A}, where h_{L} and h_{A} are the peak height of lipid and aqueous phases measured for 16-doxylstearic acid. We used 1/2 of the full height of h_{A} for valid comparison. | | | |

### Example 6

### Effect of Carboxyfullerenes on Survival in a Transgenic Model of Amyotrophic Lateral Sclerosis

One in 10,000 individuals develop ALS, the most common cause of death due to motor neuron disease. The familial form of the disease (FALS) is usually autosomal dominant, constituting 10 -15 % of cases. In 1993, a critical breakthrough identified mutations in the Cu,Zn-SOD *(sod1)* gene in certain families with FALS (Rosen et al., 1993), and subsequent work has lead to the hypothesis that ALS may be due to enhanced production of reactive oxygen species (ROS) by mutant SOD1 in some forms of FALS.

Gurney and colleagues have developed mice bearing the G⁹³A point mutation found in FALS families (Gurney et al, 1994), and the G1 line of G⁹³A transgenic mice demonstrate many of the features of human FALS. The G1 line, with 18 copies and 4 times the SOD activity of wildtype animals, develops motor neuron death by 3-4 months of age, with hindlimb weakness, impaired grooming, and thinning along their flanks. Affected mice were dead before 5 months of age (Gurney et al., 1994).

C₆₀(C(COOH)₂)₃ was used to treat FALS mice (Gurney GI strain) to determine its ability to treat FALS. At 10 weeks of age, Alzet mino-osmotic pumps (28 day, 6 (l/day, 15 mg/kg/day) containing C₆₀(C(COOH)₂)₃ (the mixed isomers, which consist of >90% O-3a) or saline were implanted intraperitoneally. The pumps were replaced at 14 weeks of age. Videotaped assessments of open field walking were performed and scored using the Bresnahan scale for spinal cord injury (Basso et al., 1995). As reported by Gurney and colleagues, these animals develop motor symptoms by approximately 90 days of age, and are moribund between 125 and 145 days of age.

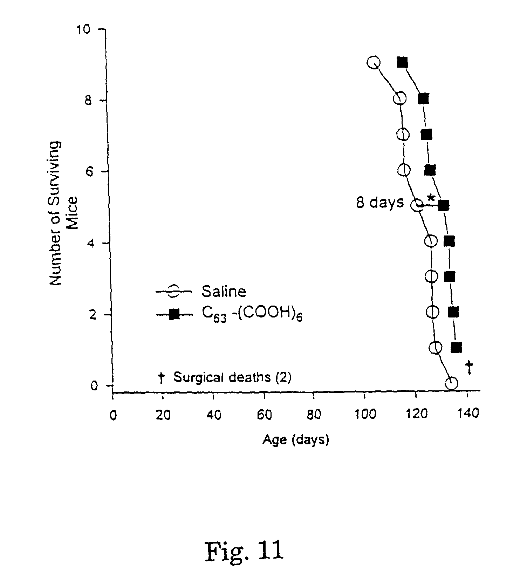

Figure 11 shows the survival curves for FALS mice treated with intraperitoneal mini-osmotic pumps containing saline or 15 mg/kg/day carboxyfullerene. These results demonstrate that the group treated with C₆₀(C(COOH)₂)₃ showed an 8±2.2 day delay in death, p = 0.041 by t-test. The FALS mice treated with C₆₀(C(COOH)₂)₃ also demonstrated a delay in onset of symptoms. The 8 day increase in survival reached statistical significance (p=0.041), by t-test. There was no difference in survival for FALS mice who received no pumps (123 days, n=4) or those treated with saline-filled pumps (125 days, n=6). In addition, wild-type animals (n=6) treated two months with carboxyfullerene (15 mg/kg/day) showed no adverse health or behavioral effects-they were as active as untreated littermates, and weights were similar (within genders).

## Claims

1. The use of carboxylated derivatives of formula
C₆₀(C(COOH)₂)ₙ I
wherein C₆₀ is buckminsterfullerene and n is 1-4
as well as pharmaceutically acceptable salts, esters and amides thereof for the manufacture of medicaments for the control or treatment of diseases caused by free radical oxygen species released by neurons due to the stimulation by Glutamate NMDA receptors.

2. The use of compounds of formula I according to claim 1, wherein the diseases include neurotoxic injury such as acute neurological insults like hypoxia/ischemia, such as occurs during stroke, hypoglycemia, epilepsy or neurodegenerative disorders such as Huntington's disease, Alzheimer's disease, amyotropic lateral sclerosis (ALS), and the neurodegenerative effects of AIDS.

3. The use according to claims 1-2, wherein in formula I n is 3.

## Patentansprüche

1. Verwendung von carboxylierten Derivaten der Formel
C₆₀(C(COOH)₂)ₙ
worin C₆₀ ein Buckminsterfulleren bedeutet und n 1 - 4 ist,
sowie pharmazeutisch annehmbare Salze, Ester und Amide davon zur Herstellung von Medikamenten für die Kontrolle oder Behandlung von Krankheiten basierend auf freien Sauerstoff-Radikalen, die ausgesendet werden durch Neuronen, welche durch den Glutamat NMDA Rezeptor stimuliert werden.

2. Die Verwendung von Verbindungen der Formel I entsprechend Anspruch 1, wobei Krankheiten zu neurotoxischen Schäden umfasst werden wie neurologische Anfälle , zum Beispiel Hypoxie/Ischämie, wie sie während eines Schlaganfalls, Hypoglykämie, Epilepsie oder neurodegenerativen Schäden wie der Huntington'schen Krankheit, der Alzheimer'schen Krankheit der Amyotrophen lateralen Sklerose (ALS) and neurodegenerativen Wirkungen von AIDS, auftreten.

3. Die Verwendung ensprechend Ansprüchen 1 - 2, worin in Formel I n 3 ist.

## Revendications

1. Utilisation de dérivés carboxylés de formule
C₆₀(C(COOH)₂)n I
dans laquelle C₆₀ représente le buckminsterfullerène et n vaut 1 - 4 ainsi que de leurs sels, esters et amides pharmaceutiquement acceptables, pour la fabrication de médicaments pour lutter contre ou traiter des maladies provoquées par des espèces oxygénées de type radicaux libres libérées par les neurones du fait de la stimulation par le glutamate des récepteurs de NMDA.

2. Utilisation des composés de formule I selon la revendication 1, dans laquelle les maladies englobent les lésions neurotoxiques telles que les accidents neurologiques aigus comme l'hypoxie/ischémie, comme cela se produit lors d'une attaque, l'hypoglycémie, l'épilepsie ou les troubles neurodégénératifs tels que la chorée de Huntington, la maladie d'Alzheimer, la sclérose latérale amyotrophique (ALS) et les effets neurodégénératifs du SIDA.

3. Utilisation selon les revendications 1-2, dans laquelle dans la formule I, n vaut 3.
